# EUROPÄISCHE PATENTANMELDUNG

(11) **EP 2 228 209 A1**
(43) Veröffentlichungstag der Anmeldung: **15.09.2010**
(21) Anmeldenummer: 09003523.9
(22) Anmeldetag: 11.03.2009
(51) Int. Cl.: B32B 5/04, A61F 13/15, B32B 3/22, B32B 27/12, B32B 37/14

(54) **Elastisches Laminat, insbesondere für elastische Windelverschlusselemente**

(71) Anmelder: Nordenia Deutschland Gronau GmbH, 48599 Gronau (DE)
(72) Erfinder: Baldauf, Georg, 48366 Laer (DE); Schönbeck, Marcus, 33775 Versmold (DE)
(74) Vertreter: Albrecht, Rainer Harald

(57) **Zusammenfassung**

Die Erfindung betrifft ein elastisches Laminat, insbesondere für elastische Windelverschlusselemente, mit Außenschichten aus Nonwoven und einer zumindest bereichsweise zwischen den Außenschichten einkaschierten elastischen Folie. Zumindest eine der beiden Außenschichten besteht aus einem in Querrichtung dehnbaren, durch Wasserstrahlen verfestigten Vliesstoff. Erfindungsgemäß ist der wasserstrahlverfestigte Vliesstoff im Bereich der elastischen Folie in zumindest einer Achsrichtung vorverstreckt.

## Beschreibung

Die Erfindung betrifft ein elastisches Laminat, insbesondere für elastische Windelverschlusselemente, mit Außenschichten aus Nonwoven und einer zumindest bereichsweise zwischen den Außenschichten einkaschierten elastischen Folie, wobei zumindest eine der beiden Außenschichten aus einem in Querrichtung dehnbaren, durch Wasserstrahlen verfestigten Vliesstoff besteht. Das Laminat wird als Materialbahn gefertigt, aus der Elemente ausgestanzt werden können, die quer zur Maschinenrichtung des Laminats elastisch sind. Aus dem elastischen Laminat können insbesondere elastische Frontpartien für Wegwerfwindeln, die auch als Windelohren bezeichnet werden, gefertigt werden.

Ein Laminat mit den beschriebenen Merkmalen ist aus EP 1 921 192 A1 bekannt. Die Außenschichten bestehen aus einem Faserflor aus Stapelfasern oder Endlosfasern, der durch eine Wasserstrahlbehandlung verfestigt worden ist. Der durch Wasserstrahlen verfestigte Vliesstoff ist mit geringer Kraft um bis zu 100 % dehnbar. Bei einer weiteren Dehnung nimmt die Dehnkraft progressiv zu, bis bei einer Dehnung von etwa 200 % die Dehngrenze erreicht ist. Die Dehngrenze ist als Begrenzung deutlich wahmehmbar.

Ausgehend von dem beschriebenen Stand der Technik liegt der Erfindung die Aufgabe zugrunde, die Dehnungseigenschaften des elastischen Laminats weiter zu verbessern.

Zur Lösung dieser Aufgabe lehrt die Erfindung, dass der wasserstrahlverfestigte Vliesstoff im Bereich der elastischen Folie in zumindest einer Achsrichtung vorverstreckt ist.

Der wasserstrahlverfestigte Vliesstoff (spunlace nonwoven), der zumindest eine der beiden Außenschichten bildet, enthält schlaufenförmige Faserstrukturen, die sich infolge der Wasserstrahlbehandlung gebildet haben. Aufgrund dieser schlaufenförmigen Strukturen, die für eine Wasserstrahlverfestigung charakteristisch sind, lässt sich das Nonwoven mit einer geringen Kraft um bis zu 100 % dehnen. Bei einer weitergehenden Dehnung nimmt die Dehnungskraft progressiv zu. Überraschenderweise zeigt sich nun, dass die Dehnungscharakteristik eines aus einer elastischen Folie und einem wasserstrahlverfestigten Vliesstoff bestehenden Kaschierverbundes durch eine Vorverstreckung des Vliesstoffes günstig beeinflusst werden kann. Je nach Art und Grad der mechanischen Vorverstreckung lassen sich unterschiedliche Effekte erzielen, die einzeln oder in Kombination die Dehnungseigenschaften des Laminats in Querrichtung vorteilhaft verändern.

Vorzugsweise ist das Laminat in Querrichtung vorverstreckt. Die Vorverstreckung erfolgt zweckmäßig durch ein Ringrollverfahren, bei dem das aus äußeren Nonwovenschichten und einer einkaschierten elastischen Folie bestehende Laminat durch eine Profilwalzenanordnung durchgeführt wird. Die Profilwalzenanordnung weist zumindest zwei ineinandergreifende Profilwalzen auf, die alternierend aus Profilwalzenabschnitten mit großen und kleinen Durchmessern zusammengesetzt sind. In der Profilwalzenanordnung wird das Laminat quer zur Laufrichtung lokal überdehnt. Durch die Eingrifftiefe der Profilwalzen ist der Verstreckungsgrad einstellbar. Bereits durch eine geringe Verstreckung des Laminats in Querrichtung kann die Dehnungscharakteristik des Laminats vorteilhaft beeinflusst werden.

Gemäß einer ersten Ausführung der Erfindung ist das Laminat durch eine Dehnung, die kleiner ist als eine durch die Bindungsstruktur des wasserstrahlverfestigten Vliesstoffes vorgegebene Dehngrenze, in Querrichtung vorverstreckt. Bevorzugt ist eine Vorverstreckung mit einem Dehnwert zwischen 100 % und 200 %. Bei einer Vorstreckung mit einem geringen Verstreckungsgrad bleibt die Bindungsstruktur des wasserstrahlvemetzten Nonwovens weitgehend erhalten. Gleichwohl wird beobachtet, dass der progressive Dehnungskraftanstieg, der für einen wasserstrahlvernetzten Vliesstoff bei einer Dehnung von mehr als 100 % charakteristisch ist, deutlich abgeschwächt ist. Im Ergebnis zeichnet sich das in der beschriebenen Weise vorgestreckte Laminat dadurch aus, dass die Dehnungskraft über einen weiten Dehnungsbereich relativ konstant bleibt. Da die Bindungsstruktur des Nonwovens bei einer Vorverstreckung von weniger als 200 % weitgehend erhalten bleibt, entspricht die Dehngrenze im Wesentlichen dem Wert eines nicht vorverstreckten Materials.

Erfindungsgemäß kann das Laminat aber auch durch eine Dehnung in Querrichtung vorverstreckt sein, die größer ist als eine durch die Bindungsstruktur des wasserstrahlverfestigten Vliesstoffes vorgegebene Dehngrenze. Durch die Vorverstreckung wird die Bindungsstruktur des wasserstrahlbenetzten Vliesstoffes verändert. Die Dehngrenze des Laminats ist in diesem Fall abhängig von der Überdehnung, mit der die Vorverstreckung ausgeführt worden ist.

Gemäß einer weiteren Ausführungsform der Erfindung wird der wasserstrahlverfestigte Vliesstoff während des Kaschiervorganges in Maschinenrichtung vorverstreckt, wobei sich das Nonwoven aufgrund der Dehnungseigenschaften in Querrichtung zusammenzieht und sich in dem Vliesstoff Wellen bilden. Der gewellte Vliesstoff wird mit der elastischen Folie verbunden. Wird das Laminat ausschließlich im Gebrauch in Querrichtung gedehnt, so werden zunächst die wellenförmigen Erhebungen des Vliesstoffes glatt gezogen. Erst dann, wenn die Wellen ihre Strecklage erreicht haben, kommen die Dehnungseigenschaften der Bindungsstruktur des wasserstrahlverfestigten Vliesstoffes zum Tragen. Beide Dehnungseffekte addieren sich, so dass das erfindungsgemäße Laminat über einen größeren Dehnungsbereich mit geringer Kraft gedehnt werden kann. Gegenstand der Erfindung ist daher auch ein elastisches Laminat, dessen Außenschicht in Form eines wasserstrahlverfestigten Vliesstoffes eine durch eine Vorverstreckung in Maschinenlaufrichtung erzeugte Struktur aufweist.

Gemäß einer bevorzugten Ausführung der Erfindung bestehen beide Außenschichten des Laminats aus einem wasserstrahlverfestigten kardiertem Vliesstoff. Der kardierte Vliesstoff kann insbesondere eine Fasermischung aus Polypropylen und Polyethylenterephthalat (PET) aufweisen, wobei der Anteil der Polypropylenfasern beliebig variiert werden kann. Bevorzugt ist eine Fasermischung aus etwa 50 % Polypropylenfasern und 50 % PET-Fasem.

Im Rahmen der Erfindung liegt es, dass eine Außenschicht aus einem wasserstrahlverfestigten Vliesstoff besteht, während die zweite Außenschicht aus einem Stapelfaservlies oder aus einem Vlies mit einem mehrschichtigen Aufbau des Typs SMS (spun-melt-spun) besteht. Die zweite Außenschicht kann thermisch oder chemisch durch Zugabe von Harzen verfestigt sein.

Zwischen den beiden Außenschichten aus Nonwoven kann eine elastische Folie flächig einkaschiert sein, die sich über die gesamte Bahnbreite erstreckt. Eine bevorzugte Ausführung der Erfindung sieht allerdings vor, dass zwischen den Außenschichten elastische Folienstreifen einkaschiert sind, wobei die Folienstreifen nebeneinander angeordnet und in Querrichtung zueinander beabstandet sind. Zwischen den elastischen Folienstreifen kann eine nicht dehnbare Verstärkungsschicht aus einem Polymer, einer Folie oder einem Spunlaid-Vliesstoff angeordnet sein, die den Abstand zwischen den elastischen Folienstreifen überbrückt und die Folienstreifen randseitig überlappt. Das auf diese Weise gebildete elastische Laminat weist elastische und nicht elastische Abschnitte auf.

Die Schichten des elastischen Laminats können thermisch beispielsweise durch punktförmige Ultraschallverschweißungen,verbunden sein. Bevorzugt ist jedoch eine Klebeverbindung. Eine bevorzugte Ausführung der Erfindung sieht vor, dass die Außenschichten aus Nonwoven mit der elastischen Folie verklebt sind, wobei der Klebstoff vollflächig, streifenförmig. gitterförmig oder punktförmig auf die zu verbindenden Flächen aufgetragen ist.

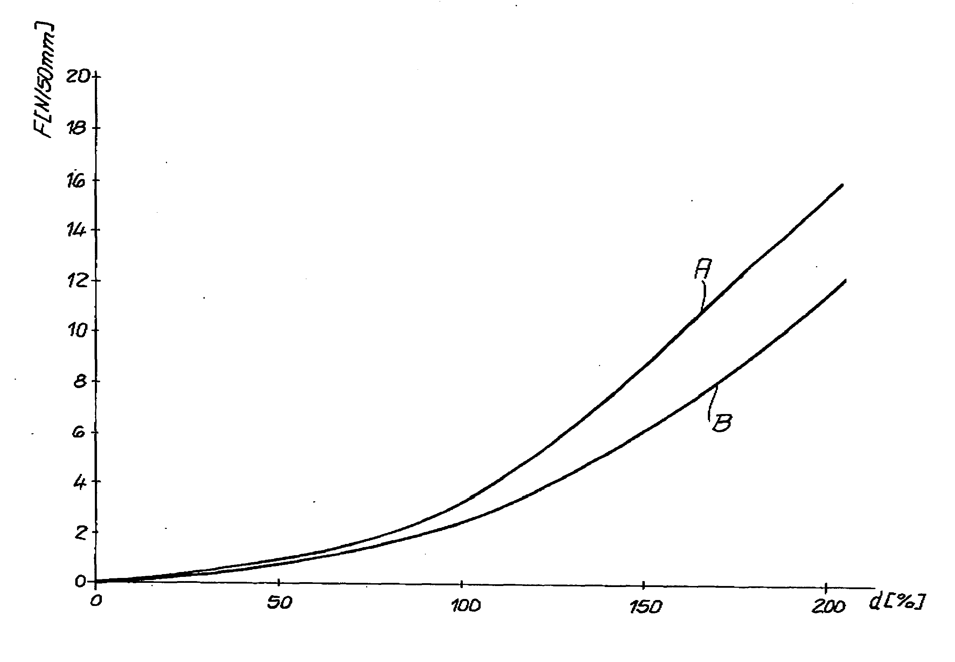

Die einzige Figur zeigt exemplarisch eine Gegenüberstellung des Dehnverhaltens eines nicht vorverstreckten elastischen Laminates mit einem erfindungsgemäßen elastischen Laminat, bei dem der wasserstrahlverfestigte Vliesstoff während des Kaschiervorganges um 20 % in Maschinenrichtung vorverstreckt ist, wobei sich der Vliesstoff aufgrund der Dehnungseigenschaften in Querrichtung zusammenzieht und sich in dem Vliesstoff Wellen bilden. Gemäß dem Ausführungsbeispiel sind das erfindungsgemäß vorverstreckte elastische Laminat und das nicht vorverstreckte elastische Laminat jeweils aus einer in ungedehntem Zustand 50 µm dicken elastischen Folie sowie beidseitig angeordneten Spunlace-Nonwovenschichten mit einem Flächengewicht von 25 g/m², die jeweils durch 7 g/m² Hotmeltkleber mit der elastischen Folie verbunden sind, gebildet. Der Verlauf B der Dehnkraft F des erfindungsgemäßen, vorverstreckten elastischen Laminates liegt in dem besonders relevanten Bereich der Dehnung d zwischen 100 und 200 % etwa 25 % unterhalb des Verlaufes A der Dehnkraft des nicht vorverstreckten Vergleichsmusters.

## Patentansprüche

1. Elastisches Laminat, insbesondere für elastische Windelverschlusselemente, mit Außenschichten aus Nonwoven und einer zumindest bereichsweise zwischen den Außenschichten einkaschierten elastischen Folie, wobei zumindest eine der beiden Außenschichten aus einem in Querrichtung dehnbaren, durch Wasserstrahlen verfestigten Vliesstoff besteht, **dadurch gekennzeichnet, dass** der durch Wasserstrahlen verfestigte Vliesstoff im Bereich der elastischen Folie in zumindest einer Achsrichtung vorverstreckt ist.

2. Elastisches Laminat nach Anspruch 1, **dadurch gekennzeichnet, dass** das Laminat in Querrichtung vorverstreckt ist.

3. Elastisches Laminat nach Anspruch 1 oder 2, **dadurch gekennzeichnet, dass** das Laminat durch eine Dehnung, die kleiner ist als eine durch die Bindungsstruktur des wasserstrahlverfesfigten Vliesstoffes vorgegebenen Dehngrenze, in Querrichtung vorverstreckt ist.

4. Elastisches Laminat nach Anspruch 3, **dadurch gekennzeichnet, dass** das Laminat durch eine Dehnung mit einem Dehnwert zwischen 100 % und 200 % vorverstreckt ist.

5. Elastisches Laminat nach Anspruch 1 oder 2, **dadurch gekennzeichnet, dass** das Laminat durch eine Dehnung, die größer ist als eine durch die Bindungsstruktur des wasserstrahlverfestigten Vliesstoffes vorgegebenen Dehnungsgrenze in Querrichtung vorverstreckt ist.

6. Elastisches Laminat nach einem der Ansprüche 1 bis 5, **dadurch gekennzeichnet, dass** der mit der elastischen Folie verbundene Wasserstrahl verfestigte Vliesstoff eine durch eine Vorverstreckung in Maschinenlaufrichtung erzeugte Struktur aufweist.

7. Elastisches Laminat nach einem der Ansprüche 1 bis 6, **dadurch gekennzeichnet, dass** beide Außenschichten des Laminats aus einem wasserstrahlverfestigten kardierten Vliesstoff bestehen.

8. Elastisches Laminat nach Anspruch 7, **dadurch gekennzeichnet, dass** der kardierte Vliesstoff eine Fasermischung aus Polypropylen (PP) und Polyethylenterephthalat (PET) aufweist.

9. Elastisches Laminat nach einem der Ansprüche 1 bis 8, **dadurch gekennzeichnet, dass** die zweite Außenschicht aus einem Stapelfaservlies besteht.

10. Elastisches Laminat nach einem der Ansprüche 1 bis 8, **dadurch gekennzeichnet, dass** die zweite Außenschicht aus einem Vlies mit einem mehrschichtigen Aufbau des Typs SMS (spun-melt-spun) besteht.

11. Elastisches Laminat nach einem der Ansprüche 1 bis 10, **dadurch gekennzeichnet, dass** zwischen den Außenschichten elastische Folienstreifen einkaschiert sind, wobei die Folienstreifen nebeneinander angeordnet und in Querrichtung zueinander beabstandet sind.

12. Elastisches Laminat nach Anspruch 11, **dadurch gekennzeichnet, dass** zwischen den elastischen Folienstreifen eine nicht dehnbare Verstärkungsschicht aus einem Polymer, einer Folie oder einem Spunlaid-Vliesstoff angeordnet ist, die den Abstand zwischen den elastischen Folienstreifen überbrückt und die Folienstreifen randseitig überlappt.

13. Elastisches Laminat nach einem der Ansprüche 1 bis 12, **dadurch geKennzeichnet, dass** die Außenschichten aus Nonwoven mit der elastischen Folie verklebt sind, wobei der Klebstoff vollflächig, streifenförmig, gitterförmig oder punktförmig auf die zu verbindenden Flächen aufgetragen ist.
